Europäisches Patentamt

European Patent Office

Office européen des brevets

(11) Veröffentlichungsnummer: **0 423 347 A1**

(12) **EUROPÄISCHE PATENTANMELDUNG**
**veröffentlicht nach Art. 158 Abs. 3**
**EPÜ**

(21) Anmeldenummer: 90902070.3

(51) Int. Cl.⁵: **A61M 5/50**

(22) Anmeldetag: 04.10.89

(86) Internationale Anmeldenummer:
**PCT/SU89/00261**

(87) Internationale Veröffentlichungsnummer:
**WO 90/12612 (01.11.90 90/25)**

(30) Priorität: 24.04.89 SU 4682845

(43) Veröffentlichungstag der Anmeldung:
**24.04.91 Patentblatt 91/17**

(84) Benannte Vertragsstaaten:
**BE CH DE FR GB IT LI SE**

(71) Anmelder: **POLTAVSKY MEDITSINSKY**
**STOMATOLOGICHESKY INSTITUT**
**ul. Shevchenko, 23,**
**Poltava 314000(SU)**

(72) Erfinder: **MAZURIK, Sergei Mikhailovich**
**ul. Lenina, 92-57**
**Poltava, 314022(SU)**
Erfinder: **EFREMOV, Oleg Viktorovich**
**ul. 60 let Oktyabrya, 3-58 Poltavskaya obl.**
**Karlovka, 315720(SU)**

(74) Vertreter: **Nix, Frank Arnold, Dr.**
**Kröckelbergstrasse 15**
**W-6200 Wiesbaden(DE)**

(54) **EINWEGSPRITZE.**

(57) Die Erfindung bezieht sich auf die Medizin.

Die Einmalspritze für Injektionen hat einen Zylinder (1), einen in diesem angeordneten Kolben (2) mit Kolbenstange (3) und einen Ansatz zum Aufsetzen der Nadel. Der Kolben (2) ist mechanisch getrennt von der Kolbenstange (3) ausgeführt; in der Kolbenstange (3) ist eine zweistufige Ringnut (5) mit einem Abschnitt (6) geringerer Tiefe und einem Abschnitt (7) größerer Tiefe ausgeführt. Auf dem Abschnitt (6) der Nut (5) ist mit Vorspannung ein Dichtring (8) aus einem elastischen Material aufgelegt, dessen Breite größer ist als der Spalt (b) zwischen dem Grund der Nut (5) auf dem Abschnitt (6) und der Innenfläche des Zylinders (1).

Die Spritze ist zur breiten Verwendung in medizinischen Anstalten und zum privaten Gebrauch bestimmt.

EP 0 423 347 A1

## EINMALSPRITZE FÜR INJEKTIONEN

Technisches Gebiet

Die Erfindung bezieht sich auf die medizinische Technik, und zwar auf Einmalspritzen, die für Injektionen verwendet werden.

Früherer Stand der Technik

Weit bekannt sind zur Zeit Einmalspritzen für Injektionen mit einem Zylinder, einen in diesem untergebrachten Kolben mit Kolbenstange und einem Ansatz zum Aufsetzen der Nadel (beispielsweise die Einmalspritzen aus der Produktion der Firma "Trumo Europe", Belgien). Diese Spritzen unterscheiden sich konstruktiv praktisch in nichts von den in der medizinischen Praxis weit bekannten, mehrmals zu verwendenden Spritzen, mit Ausnahme dessen, daß sie aus einem billigeren (polymeren) Werkstoff hergestellt sind und nicht sterilisiert werden. Dies bedeutet, daß die Konstruktion der bekannten Einmalspritzen die Möglichkeit ihrer mehrfachen Verwendung erlaubt. Dies kann geschehen aus Unaufmerksamkeit oder mangelnder Gewissenhaftigkeit des medizinischen Personals oder beim Setzen der Injektion durch Personen in einem durch Rauschgift oder Alkohol berauschten Zustand. Diese Umstände sind bedeutungsvoll, weil in diesen Fällen eine Ansteckung durch den AIDS-Virus, der infektiösen Hepatitis und anderer Krankheiten möglich ist.

Es sind auch Einmalspritzen mit einem Zylinder, einem in diesem angeordneten Kolben mit Kolbenstange und einem Ansatz zum Aufsetzen der Nadel bekannt (EP, A, 0 282 097).

Bei dieser Spritze ist die Nadel an einer Scheibe befestigt, die im vorderen Teil des Zylinders untergebracht ist und sich längs dessen Achse hin und her verschieben läßt. Der hinter der Scheibe im Spritzenzylinder angeordnete Kolben, der fest mit der Kolbenstange verbundenist, ist mit der Scheibe nicht verbunden, hat aber Greifer, die zur Kupplung des Kolbens mit der Scheibe bei deren Zusammenwirken an ihren Stirnflächen eingerichtet sind. Bei Beendigung der Injektion und bei Berührung der Stirnflächen des Kolbens und der Scheibe wird der Kolben mittels der Greifer fest mit der die Nadel tragenden Scheibe gekuppelt, sodaß bei einem wiederholten Aufziehen des Injektionsmittels in die Spritze die Scheibe mit der Nadel vom Kolben ins Innere des Spritzenzylinders eingezogen wird. Beim Eintreten der Nadel ins Innere des Zylinders kommt es zu einer Versetzung derselben bezüglich der Achse des Zylinders. Der Versuch einer wiederholten Injektion führt zum Bruch der Injektionsnadel.

Eine solche Konstruktion läßt jedoch die Möglichkeit einer wiederholten Verwendung der Spritze offen, da der Kolben erst in seiner Endstellung im vorderen Teil des Zylinders mit der Scheibe gekuppelt wird. Nur dies führt zum Einziehen der Nadel in den Zylinder, sodaß die Spritze zur weiteren Verwendung untauglich wird. Wenn der Kolben bei der Durchführung der Injektion nicht bis in seine Endlage verschoben wird, so können mit dieser Spritze beliebig viele Injektionen unter Ausnutzung fast des gesamten Rauminhaltes seines Zylinders durchgeführt werden.

Offenbarung der Erfindung

Der Erfindung liegt die Aufgabe zugrunde, eine Einmalspritze für Injektionen zu schaffen, bei der die Möglichkeit einer wiederholten Verwendung ausgeschlossen ist.

Das Wesen der Erfindung besteht darin, daß bei einer Einmalspritze für Injektionen mit einem Zylinder, einem in diesem angeordneten Kolben mit Kolbenstange und mit einem Ansatz zum Aufsetzen der Nadel erfindungsgemäß der Kolben mechanisch getrennt von der Kolbenstange ausgeführt ist, daß die Kolbenstange einen kleineren Durchmesser als der Durchmesser des Kolbens aufweist und in ihr eine zweistufige Ringnut ausgeführt ist, deren Abschnitt geringerer Tiefe zu dem dem Kolben zugewandten Ende der Kolbenstange hin gelegen ist, daß auf diesem Abschnitt der Nut ein Dichtring aus elastischem Material angeordnet ist, dessen Innendurchmesser kleiner ist als der Durchmesser der Kolbenstange an der Stelle seines Nutabschnitts geringerer Tiefe und dessen Breite größer ist als der Spalt zwischen dem Grund der Nut auf dem Abschnitt geringerer Tiefe und der Innenfläche des Zylinders.

Zweckmäßigerweise wird der Durchmesser d der Kolbenstange so gewählt, daß das Verhältnis $\frac{d}{D}$ = 0,9 beträgt, wobei D der Durchmesser des Kolbens ist.

Die Einmalspritze für Injektionen gemäß der vorliegenden Erfindung schließt die Möglichkeit der wiederholten Durchführung von Injektionen aus, sie ist einfach in der Herstellung und zuverlässig in der Anwendung. Die Aufwendungen für die Herstellung der Einmalspritze gemäß der Erfindung übersteigen praktisch nicht die Aufwendungen für die Herstellung der zur Zeit bekannten Einmalspritzen.

Kurzbeschreibung der Zeichnungen

Nachfolgend wird die Erfindung durch die Beschreibung eines konkreten Ausführungsbeispiels und die beigegebenen Zeichnungen erläutert; in diesen zeigt:
Fig. 1, 2, 3, 4 eine Einmalspritze für Injektionen gemäß der Erfindung im auseinandergenommenen Zustand in isometrischer Darstellung;
Fig. 5 die Einmalspritze für Injektionen im Ausgangszustand, Längsschnitt;
Fig. 6 das Gleiche wie in Fig. 5 beim Aufziehen des Injektionsmittels;

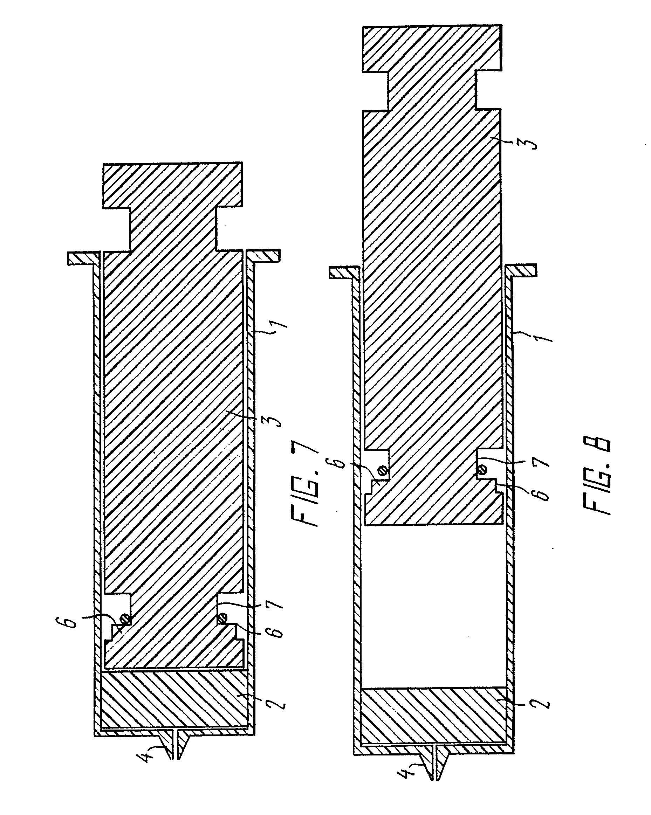

Fig. 7 das Gleiche wie in Fig. 5 nach der Durchführung der Injektion;
Fig. 8 das Gleiche wie in Fig. 5 in der Lage bei einem Versuch eines nochmaligen Aufziehens von Injektionsmittel.

Beste Verwirklichungsform der Erfindung

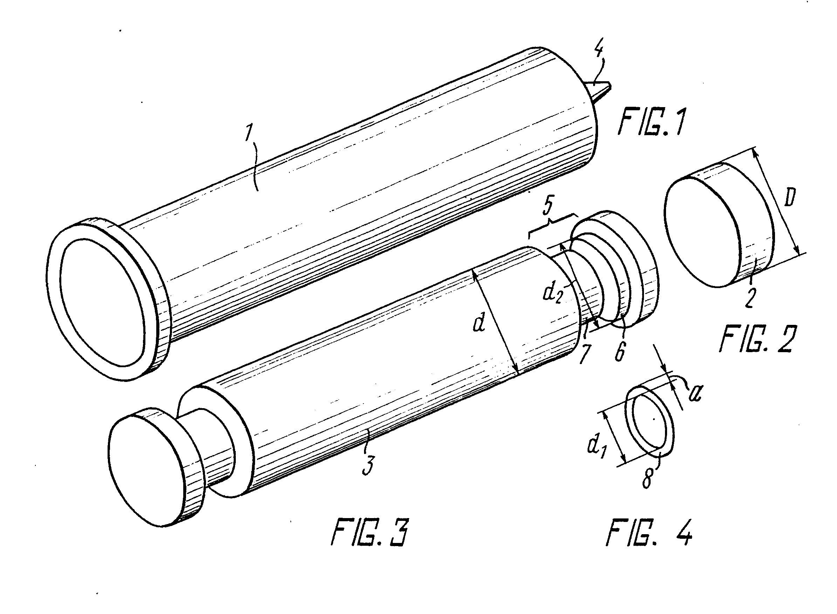

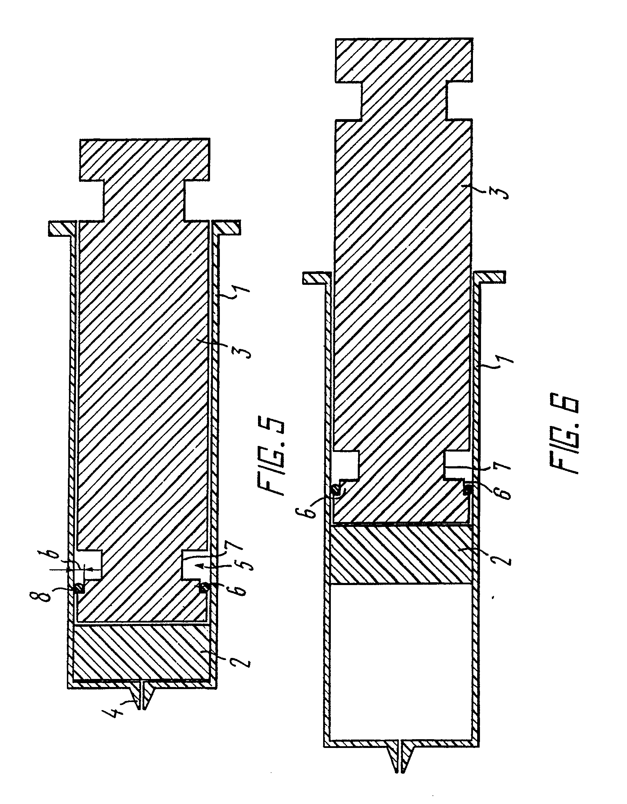

Die Einmalspritze für Injektionen gemäß der Erfindung hat einen Zylinder 1 (Fig. 1, 5), einen in diesem angeordneten Kolben 2 (Fig. 2, 5), mit Kolbenstange 3 (Fig. 3, 5). Der Zylinder 1 ist mit einem Ansatz 4 (Fig. 1, 5) zum Aufsetzen der Nadel - Kanüle - versehen, der an der Stirnseite des Vorderteils des Zylinders 1 angeordnet ist.

Der Kolben 2 ist mechanisch von der Kolbenstange 3 getrennt, wobei die Kolbenstange 3 (Fig. 3) einen Durchmesser d hat, der kleiner ist als der Durchmesser D des Kolbens 2 (Fig. 2). Wünschenswerterweise beträgt das Verhältnis $\frac{d}{D} \approx 0,9$.

In der Kolbenstange 3 (Fig. 3) ist eine zweistufige Ringnut 5 ausgeführt, die einen Abschnitt 6 geringerer Tiefe und einen Abschnitt 7 größerer Tiefe aufweist, wobei der Abschnitt 6 geringerer Tiefe näher bei dem Ende der Kolbenstange 3 (Fig. 5) liegt, die dem Kolben 2 zugewandt ist. Auf den Abschnitt 6 der Kolbenstange 3 ist ein Dichtring 8 (Fig. 4, 5) aus elastischem Werkstoff, beispielsweise Gummi, mit Vorspannung aufgesetzt. Der Innendurchmesser $d_1$ des Dichtrings 8 ist kleiner als der Durchmesser $d_2$ der Kolbenstange 3 auf dem Abschnitt 6 und die Breite a des Dichtrings 8 ist größer als der Spalt b zwischen dem Grund der Nut 5 auf dem Abschnitt 6 und der Innenfläche der Wandung des Zylinders 1. Der Dichtring 8 gewährleistet den dichten Abschluß des Raums zwischen dem Kolben 2 und der Kolbenstange 3.

Die Einmalspritze für Injektionen wird auf folgende Weise verwendet.

In der Ausgangsstellung befindet sich die Kolbenstange 3 (Fig. 5) im Zylinder 1 der Spritze. Dabei befindet sich der Kolben 2 beim vorderen Stirnteil des Zylinders 1, der den Ansatz 4 für die Injektionsnadel trägt. Nahe beim Kolben 2 befindet sich die Kolbenstange 3. Der Dichtring 8 sitzt auf dem Abschnitt 6 der Nut 5. Beim Herausziehen der Kolbenstange 3 (Fig. 6) aus dem Zylinder 1 ist der Dichtring 8 dicht an die Innenfläche des Zylinders 1 gedrückt und stützt sich auf den Vorsprung beim Abschnitt 6 der Kolbenstange 3 der Spritze. Dabei entsteht zwischen der Kolbenstange 3 und dem Kolben 2 ein Unterdruck mit der Wirkung, daß der Kolben der Kolbenstange 3 auf der gesamten Erstreckung von dessen Bewegung folgt. Auf diese Weise geschieht das Aufziehen des Injektionsmittels in den Raum des Zylinders 1 vor dem Kolben 2.

Zur Durchführung der Injektion wird die Kolbenstange 3 in den Zylinder 1 hineingeschoben. Dabei bewegt sich der Dichtring 8 auf Grund der elastischen Kräfte seines Werkstoffs, aus dem er besteht, sowie auf Grund des Unterschiedes der Durchmesser $d_1 < d_2$ in der Ringnut 5 vom Abschnitt 6 größeren Durchmessers ungehindert zum Abschnitt 7 kleineren Durchmessers. Dadurch geht die Abdichtung des Raums zwischen dem Kolben 2 und der Kolbenstange 3 verloren und nach dem Einspritzen des Injektionsstoffes verliert der Kolben 2 seine Fähigkeit, sich bei Bewegungen der Kolbenstange 3 ebenfalls zu bewegen. Dies bedeutet, daß nach einer einmaligen Einspritzung des Injektionsmittels die Spritze zu einer nochmaligen Verwendung untauglich wird, da eine Rückbewegung des Kolbens 2 nicht mehr möglich ist, weil diese durch einen Unterdruck bewirkt wird, welcher nicht mehr erzielt werden kann, wenn nicht der Dichtring 8 auf dem Abschnitt 6 der Ringnut 5 sitzt (Fig. 5).

Die Auswahl des Verhältnisses $\frac{d}{D} \approx 0,9$ macht es schwierig, den Dichtring 8 zu erreichen und unmöglich, die Spritze ohne die Hilfsmittel einer betrieblichen Fertigung in ihren Ausgangszustand (betriebsfähigen Zustand) zu versetzen.

Eine breite Anwendung der vorgeschlagenen Spritze kann praktisch eine Ansteckung des Patienten bei Injektionen mit dem AIDS-Virus, der infektiösen Hepatitis oder anderer Krankheiten verhindern, welche bei parenteraler Applikation von Heilmitteln übertragen werden.

Industrielle Anwendbarkeit

Die vorgeschlagene Spritze für Injektionen kann in beliebigen medizinischen Einrichtungen sowie beim privaten Gebrauch verwendet werden.

Ansprüche

1. Einmalspritze für Injektionen mit einem Zylinder (1), einem in diesem angeordneten Kolben (2) mit Kolbenstange (3) und einem Ansatz zum Aufsetzen der Nadel, dadurch gekennzeichnet, daß der Kolben (2) und die Kolbenstange (3) mechanisch voneinander getrennt ausgeführt sind, daß die Kolbenstange (3) mit einem kleineren Durchmesser (d) als der Durchmesser (D) des Kolbens (2) ausgeführt ist und in ihr eine zweistufige Ringnut (5) ausgeführt ist, deren Abschnitt (6) geringerer Tiefe dem dem Kolben (2) zugewandten Ende der Kolbenstange (3) näher liegt, und auf dem Abschnitt (6) der Nut (5) ein Dichtring (8) aus elastischem Werkstoff angeordnet ist, dessen Innendurchmesser ($d_1$) kleiner ist als der Durchmesser ($d_2$) der Kolbenstange (3) auf dem Abschnitt (6) der Nut (5) und dessen Breite (a) größer ist als der Spalt (b) zwischen dem Grund der Nut (5) auf dem Abschnitt (6) geringerer Tiefe und der Innenfläche des Zylinders (1).

2. Einmalspritze für Injektionen nach Anspruch 1, dadurch gekennzeichnet, daß der Durchmesser (d) der Kolbenstange (3) der Bedingung $\frac{d}{D} \approx$ 0,9 genügt, wobei D der Durchmesser des Kolbens ist.

FIG.1

FIG. 2

FIG.3

FIG. 4

EP 0 423 347 A1

FIG.5

FIG.6

FIG. 7

FIG. 8

# INTERNATIONAL SEARCH REPORT

## I. CLASSIFICATION OF SUBJECT MATTER (If several classification symbols apply, indicate all)

According to International Patent Classification (IPC) or to both National Classification and IPC

IPC$^5$: A61M 5/50

## II. FIELDS SEARCHED

### Minimum Documentation Searched[7]

| Classification System | Classification Symbols |
|---|---|
| IPC$^4$ | A61M 5/00, 5/18, 5/24, 5/28 |

Documentation Searched other than Minimum Documentation
to the Extent that such Documents are Included in the Fields Searched[8]

## III. DOCUMENTS CONSIDERED TO BE RELEVANT[9]

| Category[*] | Citation of Document,[11] with indication, where appropriate, of the relevant passages[12] | Relevant to Claim No.[13] |
|---|---|---|
| A | US, A, 4233975 (A.J. YERMAN) 18 November 1980 (18.11.80), the claims, figure 1 | 1 |
| A | US, A, 4687467 (C.T.F. RESEARCH COMPANY) 18 August 1987 (18.08.87), the claims, figure 1 | 1 |
| A | US, A, 4775363 (CHRISTIAN SANDSDALEN) 4 October 1988 (04.10.88), the claims, the drawing | 1 |

* Special categories of cited documents:[10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| 27 February 1990 (27.02.90) | 6 April 1990 (06.04.90) |

| International Searching Authority | Signature of Authorized Officer |
|---|---|
| ISA/SU | |